(19) Europäisches Patentamt
European Patent Office
Office européen des brevets

(11) EP 0 920 912 B1

(12) EUROPÄISCHE PATENTSCHRIFT

(45) Veröffentlichungstag und Bekanntmachung des Hinweises auf die Patenterteilung:
06.03.2002 Patentblatt 2002/10

(51) Int Cl.7: B01J 35/00

(21) Anmeldenummer: 98122501.4

(22) Anmeldetag: 27.11.1998

(54) **Palladium-Cluster und ihre Verwendung als Katalysatoren**

Palladium clusters and their use as catalysts

Agglomérats de palladium et leur utilisation comme catalyseurs

(84) Benannte Vertragsstaaten:
**BE DE FR GB NL**

(30) Priorität: 02.12.1997 DE 19753464

(43) Veröffentlichungstag der Anmeldung:
09.06.1999 Patentblatt 1999/23

(73) Patentinhaber: **BASF AKTIENGESELLSCHAFT**
**67056 Ludwigshafen (DE)**

(72) Erfinder:
• **Heineke, Daniel, Dr.**
**67065 Ludwigshafen (DE)**
• **Schwab, Ekkehard, Dr.**
**67434 Neustadt (DE)**
• **Fischer, Martin, Dr.**
**67071 Ludwigshafen (DE)**
• **Schmid, Guenter, Dr.**
**42555 Velbert (DE)**
• **Baeumle, Monika**
**45117 Essen (DE)**

(74) Vertreter: **Isenbruck, Günter, Dr. et al**
**Patent- und Rechtsanwälte,**
**Bardehle-Pagenberg-Dost-Altenburg-Geissler-I**
**senbruck Theodor-Heuss-Anlage 12**
**68165 Mannheim (DE)**

(56) Entgegenhaltungen:
EP-A- 0 879 642      WO-A-97/24224
DE-A- 4 443 705      DE-A- 19 506 113
US-A- 5 147 841

• **NAOKI TOSHIMA ET AL: "POLYMER-PROTECTED PALLADIUM-PLATINUM BIMETALLIC CLUSTERS: PREPARATION, CATALYTIC PROPERTIES AND STRUCTURAL CONSIDERATIONS" JOURNAL OF THE CHEMICAL SOCIETY. FARADAY TRANSACTIONS, Bd. 89, Nr. 14, 21. Juli 1993, Seiten 2537-2543, XP000381461**
• **HIRAI, HIDEFUMI; CHAWANYA, HITOSHI; TOSHIMA, NAOKI: 'Colloidal palladium protected with poly(N-vinyl-2-pyrrolidone) for selective hydrogenation of cyclopentadiene' REACTIVE POLYMERS Bd. 3, 1985, Seiten 127 - 141**

**Beschreibung**

Die Erfindung betrifft kolloides Palladium aus Palladium-Clustern, Verfahren zu seiner Herstellung, seine Verwendung, kolloides Palladium enthaltende Heterogenkatalysatoren, Verfahren zu ihrer Herstellung sowie ihre Verwendung.

Metallkolloide sind Systeme, in denen Metallteilchen mit einem Durchmesser in der Größenordnung von etwa 1 nm bis 1 μm vorliegen. Das extrem feinteilige Metall selbst wird als kolloides Metall bezeichnet. Es kann in Substanz, in einer kontinuierlichen Phase dispergiert oder an einer Phasengrenzfläche adsorbiert vorliegen. Seine Dispersion in einem Lösungsmittel wird als Metallkolloid-Lösung bezeichnet.

Unter Metall-Cluster werden Metallteilchen verstanden, die nur aus wenigen - einigen bis einigen Tausend - Metallatomen bestehen und am unteren Ende der oben angegebenen Größenskala für kolloides Metall liegen.

Die Herstellung von Metallkolloiden ist seit langem bekannt. Üblicherweise werden Metallsalze in Lösung in Gegenwart von Stabilisatoren zum Metall reduziert. Bei den Stabilisatoren handelt es sich um Substanzen, die in der Lage sind, koordinative Bindungen zum Metall einzugehen und es dadurch vor Agglomeration zu schützen. Es ist ferner bekannt, daß Eigenschaften wie Größe und Größenverteilung der gebildeten Kolloidteilchen unter anderem von der Wahl des Reduktionsmittel und des Schutzliganden, dem Verhältnis von Schutzligand zu Metallion, dem Lösungsmittel sowie dem im Metallsalz vorliegenden Anion beeinflußt werden. Eine gezielte Steuerung der Teilchengröße ist aufgrund des komplexen Bildungsmechanismus jedoch nicht möglich, vielmehr ist man auf eine empirische Optimierung angewiesen.

Metallkolloide aus Edelmetallen wie Palladium werden in großem Umfang als Katalysatoren eingesetzt. Hier sind besonders geringe Teilchengrößen wünschenswert, da bei gleicher Katalysatormenge die zur Verfügung stehende Oberfläche des Katalysators umgekehrt proportional zum Teilchendurchmesser ansteigt. Die Aktivität der Katalysatoren steht daher meist in direktem Zusammenhang mit der Größe der katalytisch aktiven Metallteilchen.

Eine Reihe von durch Metallkolloide katalysierten Reaktionen weisen zudem das Phänomen der Struktursensitivität auf, das heißt die Selektivität der Reaktion steht im Zusammenhang mit der Größe der katalytisch aktiven Teilchen. Um diesen Effekt ausnutzen zu können, müssen die Katalysatorteilchen eine enge Verteilung ihrer Teilchengröße aufweisen.

In DE-C 44 12 463 ist die Herstellung von Palladiumkolloid-Lösungen durch Reduktion von Palladiumsalzen mit einer Reihe von Reduktionsmitteln wie Phosphiten, Hypophosphiten, Boranen, Ascorbinsäure, Hydrazin und Formaldehyd in Gegenwart von Schutzkolloiden beschrieben, wobei als Schutzkolloide Polymere wie Polyvinylpyrrolidon, Polyvinylpyridin, Polyvinylmethylketon, Polyvinylalkohol, Polyvinylacetat, Polyacrylat, Alkyl- und Hydroxyalkylcellulose eingesetzt werden. Bezüglich Teilchengröße und Teilchengrößenverteilung enthält die Schrift keine Angaben. Es ist jedoch bekannt, daß nach dieser Methode hergestellte Palladiumkolloide eine sehr breite Verteilung, mit Teilchengrößen im Bereich von einigen nm bis ca. 50 nm, aufweisen.

Chinese Journal of Reactive Polymers 1 (1992), Seite 48 bis 53, offenbart die Herstellung von kolloidem Palladium durch Reduktion von Palladium(II)-chlorid in Methanol in Gegenwart von Poly-N-vinyl-2-pyrrolidon als Schutzligand mit Methanol/NaOH als Reduktionsmittel. Es werden Palladiumteilchen mit einem Durchmesser von 1 bis 3 nm und einem mittleren Durchmesser von 2 nm erhalten. Nachteilig an diesem Verfahren ist die Verwendung von Palladiumchlorid, da Chlorid als Katalysatorgift wirken kann. Auch wirken polymere Schutzliganden stark abschirmend und lassen sich nur schwer entfernen.

In J. Am. Chem. Soc. 115 (1993), Seite 2046 - 48 ist die Herstellung von kolloidem Palladium durch Reduktion von Palladium(II)acetat mit Wasserstoff in Gegenwart von Phenanthrolin als Schutzligand beschrieben. Die erhaltenen Cluster weisen zu 90% eine Teilchengröße von 3,15 und 3,6 nm auf. Das Verfahren erfordert - verfahrenstechnisch und sicherheitstechnisch aufwendig - den Einsatz von gasförmigem Wasserstoff als Reduktionsmittel.

In J. A. Chem. Soc. 116 (1994) ist die elektrolytische Herstellung von Palladium-Clustern beschrieben. Dabei wird das Edelmetall in Form einer Folie anodisch oxidiert und kathodisch zu Palladium(0) reduziert, wobei als Stabilisator leitfähige Tetraalkylammoniumsalze eingesetzt werden. Es werden Palladiumkolloide mit - je nach bei der Elektrolyse angewandter Stromdichte - variierender mittlerer Teilchengröße im Nanometer-Bereich von nm und einer relativ breiten Verteilung der Teilchengrößen erhalten. Das Verfahren macht von physiologisch bedenklichen organischen Lösungsmitteln wie Acetonitril/THF Gebrauch.

In WO97/24224 ist die Herstellung von Palladium-Clustern aus $Na_2PdCl_8 \cdot 4H_2O$ durch Reduktion mit $NaBH_4$ und Dodecylamin als Schutzligand beschrieben. Es werden Palladiumkolloide mit einer durch schnittlichen Teilchengröße von 1.8nm und einer relativ engen Teilchengrößenverteilung ($5 \leq 10\%$) erhalten.

Aufgabe der Erfindung ist es, kolloides Palladium aus Teilchen definierter Größe, insbesondere mit möglichst geringer Größe und einer engen Verteilung der Teilchengröße bereitzustellen, und dabei die oben geschilderten Nachteile zu vermeiden.

Gelöst wird die Aufgabe durch Bereitstellung von kolloidem Palladium aus Palladium-Clustern mit einem mittleren Teilchendurchmesser $0,2 \text{ nm} \leq d \leq 2 \text{ nm}$, bei dem mindestens 80 % der Palladium-Cluster einen Teilchendurchmesser aufweisen, der um höchstens 0,1 nm von dem mittleren Teilchendurchmesser abweicht, wobei die Palladium-Cluster an der Oberfläche Schutzliganden aufweisen können.

Die Aufgabe wird ferner gelöst durch ein Verfahren zur Herstellung von kolloidem Palladium aus einer Palladiumkolloid-Lösung, die durch Umsetzung eines Palladiumsalzes mit einem Reduktionsmittel in einer Lösung, enthaltend das Palladiumsalz, das Reduktionsmittel und einen Schutzliganden, bei einer Temperatur von 0 bis 300 °C, wobei als Reduktionsmittel ein verzweigtkettiger Alkohol mit 4 bis 30 C-Atomen, wobei die längste Kette 3 bis 18 C-Atome aufweist, und als Schutzliganden Phosphane und/oder aromatische Stickstoffbasen eingesetzt werden, erhalten wird.

Die Verteilung der Teilchendurchmesser der Palladium-Cluster läßt sich mathematisch durch folgende Beziehung wiedergeben:

$$x = d + 0,1 \, nm$$

$$\int [N(x)/N_{tot}] \, dx \geq 0,8$$

$$x = d - 0,1 \, nm$$

Darin bedeuten

x den Teilchendurchmesser
$N(x)$ die Zahl der Teilchen mit dem Teilchendurchmesser x
$N_{tot}$ die Gesamtzahl der Teilchen und
d den mittleren Teilchendurchmesser.

Das erfindungsgemäße kolloide Palladium weist eine besonders enge Teilchengrößenverteilung auf. So weisen mindestens 80% der erfindungsgemäßen Palladium-Cluster einen Teilchendurchmesser auf, der um höchstens 0,1 nm vom mittleren Teilchendurchmesser abweicht. Besonders bevorzugt weisen 90% der Cluster einen Teilchendurchmesser auf, der um höchstens 0,1 nm vom mittleren Teilchendurchmesser abweicht.

Die erfindungsgemäßen kolloiden Palladium-Cluster können eine oder mehrere weitere metallische Komponenten enthalten. Bevorzugte weitere metallische Komponenten sind Metalle der III. und IV. Hauptgruppe wie Gallium, Germanium, Zinn und Blei sowie Übergangsmetalle wie Re, Ru, Os, Rh, Ir, Pt, Ag und Au. Diese können in Anteilen von 0,1 bis 99 Gew.-% vorliegen.

Die erfindungsgemäßen Palladium-Cluster können an ihrer Oberfläche Schutzliganden aufweisen, wobei die Schutzliganden mit Palladium koordinative Bindungen eingehen können. Die Schutzliganden verhindern bei der Herstellung des kolloiden Palladiums durch Reduktion von Palladiumsalz in Lösung die Agglomeration der gebildeten Palladiumteilchen. Im allgemeinen weist daher unmittelbar nach der Reduktion des Palladiumsalzes zu kolloidem Palladium zumindest ein Teil der Palladium-Cluster an der Oberfläche adsorbierte Schutzliganden auf. Auch in späteren Verarbeitungsstufen - beispielsweise nach Isolierung des kolloiden Palladiums aus der gebildeten Palladiumkolloid-Lösung und gegebenenfalls Redispergierung in einem flüssigen Medium, oder auch nach Adsorption der Cluster an einen Träger - können die Palladium-Cluster an ihrer Oberfläche noch Schutzliganden aufweisen. Es ist jedoch möglich, die Schutzliganden von der Oberfläche der Cluster zu entfernen. Im allgemeinen sind dazu besondere Maßnahmen wie eine Wärmebehandlung des kolloiden Palladiums erforderlich.

Die Herstellung des kolloiden Palladiums erfolgt in der Weise, daß zunächst eine Palladiumkolloid-Lösung hergestellt wird. Aus dieser kann das kolloide Palladium isoliert werden. Die Herstellung der Palladiumkolloid-Lösung erfolgt durch Umsetzung eines Palladiumsalzes in Lösung mit einem Reduktionsmittel in Gegenwart eines Schutzliganden. Es wurde gefunden, daß unter Verwendung von verzweigtkettigen Alkoholen als Reduktionsmittel kolloides Palladium mit einer sehr engen Verteilung der Teilchengröße erhalten wird.

Bevorzugte Reduktionsmittel sind unverzweigt- und verzweigtkettige Alkohole mit 4 bis 30 C-Atomen, wobei die längste Kette 3 bis 18 C-Atome aufweist. Bevorzugte Alkohole weisen einen Siedepunkt von 50 bis 300 °C auf. Besonders bevorzugte Reduktionsmittel weisen 1 bis 3 Verzweigungsstellen und eine längste Kette mit 3 bis 18 C-Atomen auf, von diesen sind die primären Alkohole mit nur einer Verzweigungsstelle insbesondere bevorzugt. Beispiele sind 2-Methylpropanol, 2- und 3-Methylbutanol, 2-Ethylbutanol, 2-, 3- und 4-Methylpentanol, 2- und 3-Ethylpentanol sowie 2-Propylpentanol. Speziell bevorzugt ist 3-Methylbutanol.

Zur Herstellung sind prinzipiell alle Palladiumsalze einsetzbar, im allgemeinen werden Palladium(II) salze eingesetzt. Wegen des späteren Einsatzes der gebildeten Palladium-Cluster als Katalysatoren sollten die Anionen keine Bestandteile enthalten, die als Katalysatorgift wirken können. Aus diesem Grund sind beispielsweise Palladium(II)chlorid und Palladium(II)sulfat weniger bevorzugt. Bevorzugt sind hingegen Palladium (II)nitrat und Palladium(II)salze von Carbonsäuren, besonders bevorzugt ist Palladium(II)acetat.

Um Palladium-Cluster mit weiteren metallischen Komponenten zu erhalten, können entsprechende Metallsalze wie die Salze der oben genannten Metalle der III und IV Hauptgruppe und Übergangsmetalle mitverwendet werden.

Als Schutzliganden sind prinzipiell solche Verbindungen geeignet, die mit Palladium koordinative Bindungen eingehen können. Bevorzugt sind niedermolekulare Verbindungen, da sich diese später leichter entfernen lassen. Insbesondere kommen als Schutzliganden Phosphane und aromatische Stickstoffverbindungen in Frage. Aromatische Stickstoffverbindungen im Sinne dieser Erfindung sind aromatische Verbindungen, in denen das aromatische System mindestens ein Stickstoffatom aufweist.

Als Schutzliganden werden bevorzugt unter den Reaktionsbedingungen stabile Phosphane und aromatische Stickstoffverbindungen eingesetzt. Bevorzugte Phosphane sind solche der allgemeinen Formel $PR^1R^2R^3$, wobei $R^1$, $R^2$, $R^3$, die gleich oder verschieden sein können, für Phenyl und Cyclohexyl stehen, wobei die genannten Reste Substituenten tragen können. Als Substituenten kommen insbesondere die Wasserlöslichkeit der Phosphane erhöhende Gruppen wie Sulfonsäure- und Aminogruppen in Frage, die bevorzugt an einen Phenylrest gebunden sind. Das Vorliegen solcher Substituenten ist insbesondere dann von Vorteil, wenn in einem wäßrigen Lösungsmittel gearbeitet oder die Wasserdispergierbarkeit der Palladium-Cluster erhöht werden soll. Beispiele sind an einem oder mehreren Phenylringen sulfonierte Triphenylphosphane der allgemeinen Formel $P(C_6H_5SO_3M)_{3-x}(C_6H_5)_x$ mit x = 0, 1, 2 und M = Alkalimetall. Bevorzugte aromatische Stickstoffbasen sind Pyridine, Bipyridine und Phenanthroline, die am aromatischen Ring mit $C_1$-$C_{18}$-Alkyl, $C_5$-$C_{10}$-Cycloalkyl, Halogen, Hydroxyl, $C_1$-$C_6$-Alkoxy sowie Amino-, Monoalkyl- bzw. Dialkylamino substituiert sein können. Besonders bevorzugte Schutzliganden sind Triphenylphosphane, insbesondere Triphenylphosphan, sowie Phenanthroline, insbesondere 1,10-Phenanthrolin, und Bipyridine.

Das Gewichtsverhältnis Palladium:Schutzligand beträgt während der Synthese im allgemeinen von 100:1 bis 1:1000, bevorzugt von 10:1 bis 1:100, besonders bevorzugt von 5:1 bis 1:15. Das optimale Verhältnis Palladium:Schutzligand hängt auch von der Art des verwendeten Schutzliganden ab. Es kann sich, wie die Wahl des Schutzliganden, auf die Größe der gebildeten Cluster auswirken. Bei den oben genannten besonders bevorzugten Schutzliganden beträgt es insbesondere von 5:1 bis 1:15, speziell von 1:4 bis 1:13.

Für die Synthese der Palladium-Cluster sind polare wie unpolare Lösungsmittel geeignet. Es kann beispielsweise in einem wäßrigen Lösungsmittel gearbeitet werden, das das Reduktionsmittel gelöst enthält. Dabei kann als Schutzligand das vorstehend genannte sulfonierte Triphenylphosphan eingesetzt werden. Es kann aber auch in nichtwäßrigen Lösungsmitteln, die das Reduktionsmittel enthalten, gearbeitet werden. Beispiele sind Alkohole, Essigsäure, THF sowie Ether. In einer bevorzugten Ausführungsform der Erfindung wird in dem Reduktionsmittel als Lösungsmittel gearbeitet, insbesondere wird dazu 3-Methylbutanol eingesetzt.

Die Umsetzung des Palladiumsalzes mit dem Reduktionsmittel erfolgt im allgemeinen durch Rühren einer Lösung enthaltend das Palladiumsalz, ggf. ein weiteres Metallsalz, den Schutzliganden sowie das Reduktionsmittel bei einer Umsetzungstemperatur von 0 bis 300 °C , bevorzugt 20 bis 80 °C, besonders bevorzugt 50 bis 70 °C, und über einen Zeitraum von einigen Minuten bis einigen Tagen, bevorzugt 2 bis 10 Tage, besonders bevorzugt 2 bis 7 Tage. Die Umsetzungstemperatur hängt unter anderem vom verwendeten Lösungsmittel und Reduktionsmittel ab, die Reaktionsdauer richtet sich unter anderem nach dem Schutzliganden.

Aus der nach dem vorstehend beschriebenen Verfahren hergestellten Palladiumkolloid-Lösung kann das kolloide Palladium durch Zusatz eines sehr unpolaren Lösungsmittels gefällt und isoliert werden. Geeignete sehr unpolare Lösungsmittel sind beispielsweise aliphatische, aromatische oder cycloaliphatische Kohlenwasserstoffe mit 5 bis 10 C-Atomen. Insbesondere hat sich der Zusatz von Petrolether als Fällungsmittel bewährt. Das gefällte kolloide Palladium kann durch übliche mechanische Abtrennverfahren isoliert werden, beispielsweise durch Filtrieren oder Zentrifugieren. Auch in Substanz sind die erfindungsgemäßen Palladium-Cluster luftstabil, so daß sie nach der Isolierung an der Luft getrocknet werden können.

Das erfindungsgemäße kolloide Palladium kann als Hydrierungskatalysator verwendet werden. Bevorzugte Hydrierungsreaktionen sind die Hydrierung von Olefinen, Acetylenen, Dienen, gesättigten und ungesättigten Nitrilen. Dazu kann das isolierte kolloide Palladium in einem flüssigen Medium zu einer Palladiumkolloid-Lösung redispergiert werden. Es können auch die bei der Reduktion des Palladiumsalzes erhaltene Palladiumkolloid-Lösungen direkt eingesetzt werden. Das erfindungsgemäße kolloide Palladium kann ferner auf einen Träger aufgebracht werden.

Bevorzugt wird das erfindungsgemäße kolloide Palladium zu einem palladiumhaltigen Heterogenkatalysator weiterverarbeitet, indem es auf einen Träger aufgebracht wird. In Frage kommen alle üblichen Träger wie keramische Oxide und Kohlenstoff, bevorzugt $Al_2O_3$ wie γ- oder δ-$Al_2O_3$, $SiO_2$, $ZrO_2$, $TiO_2$ (Rutil, Anatas) und deren Mischoxiden, Kohlenstoff (außer Diamant), Zeolithe und Silikalite wie Titansilikalit. Die Träger können Promotoren zur Erhöhung der katalytischen Aktivität und der Sinterstabilität enthalten. Geeignete Promotoren sind Alkali- und Erdalkalimetalle, Lanthanide und Actinide. Bevorzugte Träger sind Aktivkohle, Siliziumdioxid, Aluminiumoxid Titandioxid und Zeolithe.

Das kolloide Palladium kann aus Lösung auf den Träger aufgebracht werden. Dazu wird der Träger mit der Palladiumkolloid-Lösung imprägniert. Das Imprägnieren kann in an sich bekannter Weise durch Tränken des Trägers in der Lösung oder Besprühen mit der Lösung erfolgen. Nach dem Imprägnieren kann sich ein Trocknungsschritt anschließen. Die so hergestellten Heterogenkatalysatoren können direkt zur Synthese eingesetzt werden oder in einem weiteren Schritt calciniert werden. Beim Calcinieren können die Schutzliganden abgespalten werden, ohne daß sich die Teilchengrößen der Palladium-Cluster verändern. Das Calcinieren findet im allgemeinen bei Temperaturen von 100 bis 600 °C, bevorzugt von 120 bis 400 °C, besonders bevorzugt von 150 bis 200 °C statt.

Das kolloide Palladium kann aber auch durch Trockenvermischung des in Substanz isolierten kolloiden Palladiums mit dem Träger auf diesen aufgebracht werden. Es kann sich ebenfalls ein Calcinierungsschritt anschließen.

Der Palladiumgehalt der Heterogenkatalysatoren beträgt im allgemeinen 0,001 bis 10 Gew.-%, bevorzugt 0,01 bis 7 Gew.-%, besonders bevorzugt 0,03 bis 5 Gew.-% und richtet sich nach den mit diesen Katalysatoren durchzuführenden Reaktionen. Die erfindungsgemäßen palladiumhaltigen Heterogenkatalysatoren eigenen sich wie das kolloide Palladium selbst für katalytische Hydrierungen, insbesondere für die oben genannten Hydrierungsreaktionen.

Dazu wird beispielsweise der palladiumhaltige Heterogenkatalysator in dem zu hydrierenden Olefin, Acetylen, Dien oder Nitril und gegebenenfalls einem zusätzlichen Lösungsmittel dispergiert und es wird bei 1 bis 100 bar, vorzugsweise 1 bis 10 bar Wasserstoffdruck hydriert.

Die Erfindung wird durch die nachstehenden Beispiele näher erläutert.

**Beispiel 1**

Herstellung von Palladium-Clustern mit Triphenylphosphan als Schutzligand

2,95 g Palladium(II)acetat und 5,6 g Triphenylphosphan werden unter Stickstoff in 1 1 3-Methylbutanol gelöst und anschließend 2 Tage bei 60 °C unter Rückfluß gerührt. Nach dem Abkühlen wird das Reaktionsgemisch mit der 5-fachen Menge an Petrolether versetzt und die Suspension 30 min bei 5000 Umdrehungen/min zentrifugiert. Die überstehende, bräunlich gefärbte Lösung wird abdekantiert und das Produkt an der Luft getrocknet. Zur Redispergierung wird der schwarzgefärbte Feststoff mit einer Mischung aus Wasser und Pyridin (1:1) versetzt.

Aus der Transmissionselektronenmikroskop-Aufnahme wurde die Größenverteilung des kolloiden Palladiums durch Ausmessen und Zählen der einzelnen Cluster bestimmt. Danach weisen die Palladium-Cluster in Grenzen einen einheitlichen Teilchendurchmesser von ca. 1,3 nm auf. Die Abweichungen liegen unterhalb der Meßgenauigkeit von ca. 0,1 nm.

**Beispiel 2**

Herstellung von Palladium-Clustern mit Phenanthrolin als Schutzligand

300 mg Palladium(II)acetat und 1900 mg 1,10-Phenanthrolin werden unter Stickstoff in 100 ml 3-Methylbutanol gelöst. Die Lösung wird 7 Tage bei 60 °C unter Rückfluß erhitzt. Nach dem Abkühlen wird die Lösung mit ca. der 4-fachen Menge an Petrolether versetzt und bei 5000 U/min zentrifugiert. Die überstehende farblose Lösung wird abdekantiert und der Rückstand an der Luft getrocknet. Zur Redispergierung wird der schwarze Feststoff mit einer Mischung aus Wasser und Pyridin (1:1) versetzt.

Laut TEM-Aufnahme weisen ca. 80 % der Palladium-Cluster den gleichen Teilchendurchmesser von ca. 1,6 nm auf.

**Beispiel 3**

Herstellung eines palladiumhaltigen Heterogenkatalysators

Eine gemäß Beispiel 2 hergestellte Palladiumkolloid-Lösung wird zu im gleichen Lösungsmittel suspendierter Aktivkohle (Degusorb® von Degussa) zugetropft. Nach quantitativer Adsorption, erkennbar an der vollständigen Entfärbung der Lösung, wird filtriert und anschließend im Vakuum getrocknet.

Die katalytische Aktivität der heterogenisierten Palladium-Cluster wurde anhand der katalytischen Hydrierung von 2-Hexin überprüft.

**Beispiel 4**

100 mg des Katalysators aus Beispiel 3 werden in 25 ml 2-Hexin suspendiert. Die Suspension wird bei 25 °C und 1 bar mit Wasserstoff gesättigt und gerührt. Nach einer Reaktionszeit von 2 h sind 81% des Eduktes umgesetzt, wobei mit einer Selektivität von 100% die Hydrierung zu cis-2-Hexen erfolgt.

**Beispiel 5**

Es werden 100 mg des Heterogenkatalysators aus Beispiel 3 bei 180 °C über einen Zeitraum von ca. 5 Stunden calciniert. Der calcinierte Katalysator wird in 25 ml 2-Hexin suspendiert und bei 25 °C und 1 bar mit Wasserstoff gesättigt und gerührt. Nach 2 h sind 99 % des Edukts mit einer Selektivität von 100 % zu cis-2-Hexen umgesetzt.

**Patentansprüche**

1. Kolloides Palladium aus Palladium-Clustern mit einem mittleren Teilchendurchmesser $0,2 \text{ nm} \leq d \leq 2 \text{ nm}$, **dadurch gekennzeichnet, dass** mindestens 80% der Palladium-Cluster einen Teilchendurchmesser aufweisen, der um höchstens 0,1 nm von dem mittleren Teilchendurchmesser d abweicht, wobei die Palladium-Cluster an der Oberfläche Schutzliganden aufweisen können.

2. Kolloides Palladium nach Anspruch 1, **dadurch gekennzeichnet, daß** zumindest ein Teil der Palladium-Cluster an der Oberfläche Schutzliganden aufweist, wobei die Schutzliganden mit Palladium koordinative Bindungen eingehen können.

**3.** Kolloides Palladium nach Anspruch 2, **dadurch gekennzeichnet, daß** die Schutzliganden Phosphane und/oder aromatische Stickstoffverbindungen sind.

**4.** Kolloides Palladium nach einem der Ansprüche 2 oder 3, **dadurch gekennzeichnet, daß** die Schutzliganden Triphenylphosphane, Phenanthroline oder Bipyridine sind.

**5.** Palladiumkolloid-Lösung, enthaltend kolloides Palladium nach einem der Ansprüche 1 bis 4.

**6.** Verfahren zur Herstellung einer Palladiumkolloid-Lösung, wie sie in Anspruch 5 definiert ist, durch Umsetzung eines Palladiumsalzes mit einem Reduktionsmittel in einer Lösung, enthaltend das Palladiumsalz, das Reduktionsmittel und einen Schutzliganden, bei einer Temperatur von 0 bis 300 °C, **dadurch gekennzeichnet, daß** als Reduktionsmittel ein verzweigtkettiger Alkohol mit 4 bis 30 C-Atomen, wobei die längste Kette 3 bis 18 C-Atome aufweist, und als Schutzliganden Phosphane und/oder aromatische Stickstoffverbindungen eingesetzt werden.

**7.** Verfahren zur Herstellung von kolloidem Palladium mit den Schritten:

a) Herstellung einer Palladiumkolloid-Lösung gemäß einem Verfahren nach Anspruch 6;
b) Fällung des kolloiden Palladiums durch Zugabe eines Kohlenwasserstoff-Lösungsmittels;
c) mechanisches Abtrennen des kolloiden Palladiums;
d) Trocknen des kolloiden Palladium.

**8.** Verwendung von kolloidem Palladium, wie in einem der Ansprüche 1 bis 4 definiert, oder einer Palladiumkolloid-Lösung, wie in Anspruch 5 definiert, zur Hydrierung von Olefinen, Acetylenen, Dienen sowie gesättigten und ungesättigten Nitrilen.

**9.** Palladiumhaltiger Heterogenkatalysator, enthaltend auf einem Träger aufgebrachtes kolloides Palladium gemäß einem der Ansprüche 1 bis 4.

**10.** Verfahren zur Herstellung eines palladiumhaltigen Heterogenkatalysators, wie er in Anspruch 9 definiert ist, mit den Schritten:

a) Herstellung einer Palladiumkolloid-Lösung gemäß dem Verfahren nach Anspruch 6;

b) Imprägnieren eines Trägers, ausgewählt aus der Gruppe $Al_2O_3$, $SiO_2$, $ZrO_2$, $TiO_2$ und deren Mischoxide, Kohlenstoff, Zeolithe und Silikalite mit der Palladiumkolloid-Lösung;

c) Trocknen des imprägnierten Trägers, der anschließend calciniert werden kann.

**11.** Verfahren nach Anspruch 10, **dadurch gekennzeichnet, daß** das Imprägnieren des Trägers durch Vermischen der Palladiumkolloid-Lösung mit einer Suspension des Trägers im gleichen Lösungsmittel erfolgt und der Träger anschließend mechanisch abgetrennt wird.

**12.** Verfahren zur Herstellung eines palladiumhaltigen Heterogenkatalysators durch Trockenvermischung von kolloidem Palladium, wie es in einem der Ansprüche 1 bis 4 definiert ist, mit einem Träger, wie er in Anspruch 10 definiert ist, wobei die erhaltene Mischung anschließend calciniert werden kann.

**13.** Verwendung des palladiumhaltigen Heterogenkatalysators, wie er in Anspruch 9 definiert ist, zur Hydrierung von Olefinen, Acetylenen, Dienen sowie gesättigten und ungesättigten Nitrilen.

**Claims**

**1.** Colloidal palladium composed of palladium clusters with an average particle diameter 0.2 nm # d # 2 nm, wherein at least 80% of the palladium clusters have a particle diameter which differs by not more than 0.1 nm from the average particle diameter d, it being possible for the palladium clusters to have protective ligands on the surface.

**2.** Colloidal palladium as claimed in claim 1, wherein at least some of the palladium clusters have protective ligands on the surface, the protective ligands being able to coordinate with palladium.

**3.** Colloidal palladium as claimed in claim 2, wherein the protective ligands are phosphines and/or aromatic nitrogen compounds.

**4.** Colloidal palladium as claimed in either of claims 2 or 3, wherein the protective ligands are triphenylphosphanes, phenanthrolines or bipyridines.

**5.** A colloidal solution of palladium comprising colloidal palladium as claimed in any of claims 1 to 4.

**6.** A process for preparing a colloidal solution of palladium as defined in claim 5 by reacting a palladium salt with a reducing agent in a solution comprising the palladium salt, the reducing agent and a protective ligand at from 0 to 300°C, wherein a branched-chain alcohol having 4 to 30 carbon atoms, where the longest chain has 3 to 18 carbon atoms, is employed as reducing agent, and phosphines and/or aromatic nitrogen compounds are employed as protective ligands.

7. A process for preparing colloidal palladium, comprising the steps:

> a) preparing a colloidal solution of palladium by a process as claimed in claim 6;
> b) precipitating the colloidal palladium by adding a hydrocarbon solvent;
> c) mechanically removing the colloidal palladium;
> d) drying the colloidal palladium.

8. The use of colloidal palladium as defined in any of claims 1 to 4 or of a colloidal solution of palladium as defined in claim 5 for the hydrogenation of olefins, acetylenes, dienes and saturated and unsaturated nitriles.

9. A palladium-containing heterogeneous catalyst comprising colloidal palladium as claimed in any of claims 1 to 4 applied to a carrier.

10. A process for preparing a palladium-containing heterogeneous catalyst as defined in claim 9, comprising the steps:

> a) preparing a colloidal solution of palladium by a process as claimed in claim 6;
> b) impregnating a carrier selected from the group of $Al_2O_3$, $SiO_2$, $ZrO_2$, $TiO_2$ and mixed oxides thereof, carbon, zeolites and silicalites with the colloidal solution of palladium;
> c) drying the impregnated carrier, which can then be calcined.

11. A process as claimed in claim 10, wherein the carrier is impregnated by mixing the colloidal solution of palladium with a suspension of the carrier in the same solvent, and the carrier is subsequently removed mechanically.

12. A process for preparing a palladium-containing heterogeneous catalyst by mixing colloidal palladium as defined in any of claims 1 to 4 dry with a carrier as defined in claim 10, the resulting mixture possibly being subsequently calcined.

13. The use of the palladium-containing heterogeneous catalyst as defined in claim 9 for the hydrogenation of olefins, acetylenes, dienes and saturated and unsaturated nitriles.

**Revendications**

1. Palladium colloïdal constitué d'agglomérats de palladium avec des particules d'un diamètre moyen de $0,2 \, nm \leq d \leq 2 \, nm$, **caractérisé en ce que** 80 % des agglomérats de palladium présentent des particules d'un diamètre qui diffère au maximum de 0,1 nm du diamètre moyen des particules d, dans lequel cas les agglomérats de palladium peuvent présenter des ligands protecteurs sur leur surface.

2. Palladium colloïdal selon la revendication 1, **caractérisé en ce qu'**au moins une partie des agglomérats de palladium présente des ligands protecteurs sur sa surface, dans lequel cas les ligands protecteurs peuvent contracter des liaisons datives avec le palladium.

3. Palladium colloïdal selon la revendication 2, **caractérisé en ce que** les ligands protecteurs sont des phosphanes et/ou des composés azotés aromatiques.

4. Palladium colloïdal selon l'une quelconque des revendications 2 ou 3, **caractérisé en ce que** les ligands protecteurs sont des triphénylphosphanes, de la phénanthroline ou de la bipyridine.

5. Solution de colloïde de palladium, contenant du palladium colloïdal selon l'une quelconque des revendications 1 à 4.

6. Procédé de fabrication d'une solution de colloïde de palladium, telle qu'elle est définie sous la revendication 5, par transformation d'un sel de palladium à l'aide d'un agent de réduction dans une solution contenant le sel de palladium, l'agent de réduction et un ligand protecteur, à une température située entre 0 et 300 °C, **caractérisé en ce que** l'agent de réduction est un alcool à chaîne ramifiée avec 4 à 30 atomes C, dans lequel cas la chaîne la plus longue présente de 3 à 18 atomes C et **en ce que** les ligands protecteurs utilisés sont des phosphanes et/ ou des composés azotiques aromatiques.

7. Procédé de fabrication de palladium colloïdal comprenant les étapes suivantes :

> a) fabrication d'une solution de colloïde de palladium selon un procédé selon la revendication 6 ;
> b) précipitation du palladium colloïdal par addition d'un solvant à base d'hydrocarbure ;
> c) séparation mécanique du palladium colloïdal ;
> d) séchage du palladium colloïdal

8. Utilisation de palladium colloïdal selon la définition de l'une quelconque des revendications 1 à 4 ou d'une solution de colloïde de palladium selon la définition de la revendication 5, pour l'hydrogénation d'oléfines, d'acétylènes, de dioléfines ainsi que de nitriles saturés ou insaturés.

**9.** Catalyseur hétérogène contenant du palladium, sous forme de palladium colloïdal appliqué sur un porteur selon l'une quelconque des revendications 1 à 4.

**10.** Procédé de fabrication d'un catalyseur hétérogène contenant du palladium, tel qu'il est défini sous la revendication 9, comprenant les étapes suivantes :

a) fabrication d'une solution de colloïde de palladium selon le procédé explicité sous la revendication 6 ;
b) imprégnation d'un porteur, sélectionné dans le groupe $Al_2O_3$, $SiO_2$, $ZrO_2$, $TiO_2$ et leurs oxydes mixtes, le carbone, les zéolites et les silicalites, avec la solution de colloïde de palladium.
c) séchage du porteur imprégné, qui peut ensuite être calciné.

**11.** Procédé selon la revendication 10, **caractérisé en ce que** l'imprégnation du porteur est réalisée par mélange de la solution de colloïde de palladium avec le porteur en suspension dans le même solvant et **en ce que** le porteur est ensuite séparé mécaniquement.

**12.** Procédé de fabrication d'un catalyseur hétérogène contenant du palladium par mélange à sec de palladium colloïdal, selon les définitions des revendications 1 à 4, avec un porteur selon la définition de la revendication 10, dans lequel cas le mélange obtenu peut ensuite être calciné.

**13.** Utilisation du catalyseur hétérogène contenant du palladium, selon la définition de la revendication 9, pour l'hydrogénation d'oléfines, d'acétylènes, de dioléfines ainsi que de nitriles saturés ou insaturés.